# EUROPEAN PATENT APPLICATION

(11) **EP 1 738 769 A1**
(43) Date of publication of application: **03.01.2007**
(21) Application number: 05727376.5
(22) Date of filing: 31.03.2005
(51) Int. Cl.: A61K 47/36, C08B 37/00, C08B 37/02, C12N 15/09

(54) **PREPARATION FOR TRANSFERRING NUCLEIC ACID INTO CELL**

(30) Priority: 31.03.2004 JP 2004103568
(71) Applicant: Medgel Corporation, Kyoto-shi, Kyoto 612-8043 (JP)
(72) Inventor: TABATA, Yasuhiko, 6110024 (JP)
(74) Representative: Vossius & Partner
(86) International application number: PCT/JP2005/006370
(87) International publication number: WO 2005/094894

(57) **Abstract**

The present invention provides a composition comprising a polysaccharide to enhance transfection of a cell with a nucleic acid. The present invention also provides a method for transfecting a cell with a nucleic acid comprising the steps of forming a polyionic complex of the nucleic acid and a polysaccharide; and bringing about uptake of the polyionic complex by the cell. Preferably, the cell is selected from bone marrow mesenchymal stem cells, nervous system cell lines, adipose tissue stem cells, immunocytes, neurons, and chondrocytes. Moreover, preferably the polysaccharide is a cationized pullulan derivative, a cationized dextran derivative, or a cationized mannan derivative.

## Description

### TECHNICAL FIELD

The present invention relates to a pharmaceutical preparation comprising a polysaccharide for transfecting a cell with a nucleic acid.

### BACKGROUND ART

Technology for transfecting a cell with a nucleic acid and increasing the expression thereof is important not only for basic biomedical research, but also for gene therapy, genetic modification of cells for cell transplantation therapy, regenerative medicine, and the like. Previous methods for transfecting a cell with a nucleic acid are roughly divided into methods using viruses and methods not using viruses. For their own survival viruses easily enter cells passively or have properties that encourage active uptake by cells. By using this property the intracellular uptake of nucleic acids can be achieved using a virus as a carrier, and a high uptake efficiency can be obtained thereby. However, there are problems associated with the use of viruses, such as immunogenicity, toxicity, and the like. Thus, transfection of a cell with a nucleic acid by methods that do not use a virus have been attempted.

The uptake of a substance by a cell occurs by two mechanisms. First, a cell regularly takes in liquid (pinocytosis), and substances in liquids surrounding the cell are also taken into the cell thereby. The second is an uptake mechanism via a receptor on the surface of the cell that recognizes and takes up a specific molecule. It is known that the latter provides a significantly higher substance uptake efficiency than the former.

An example of pinocytosis involves the use of a cationized polymer, liposome, and the like as a nucleic acid carrier. This brings about an interaction between negatively charged nucleic acids and cells with a negatively charged surface, and the basic idea is to neutralize the negative charge of the nucleic acid by a polyionic complex and decrease the size of the molecule containing the same so that it will be incorporated intracellularly. High efficiency cannot be expected with this method, however, because intracellular uptake depends on the pinocytosis mechanism. There are also the calcium phosphate method and the like, but these methods basically involve the same mechanism as the cationized carrier, and efficiency is also low.

Thus, enhancing the permeability of the cell membrane by various types of physical stimulation has been attempted to increase the uptake efficiency. For example, improvement in uptake efficiency has been reported using electroporation, sonoporation, and the like, i.e., electrical stimulation and irradiation with ultrasonic waves. The use of a gene gun, microinjection, and the like have also been reported. These attempts demonstrate efficacy in increasing uptake to a certain extent, but this is not true for all cells. More specifically, these methods demonstrate almost no efficacy in stem and progenitor cells in embryos and tissues that have superb growth and differentiation capabilities, or in cells that have specific biological functions such as immunocytes, neurons, chondrocytes and the like. Additionally, even if the nonviral cationized polymer or liposome carrier noted above is used, intracellular transfer of the nucleic acid is not easily achieved.

Therefore, developmental research into nonviral carriers appears to be headed toward the use of cell surface receptors, which have greater uptake efficiency than pinocytosis. These receptors can be roughly divided into two types. The first type recognizes specific amino acid sequences of proteins, and the other type recognizes sugar molecules.

There have been many studies concerning the promotion of intracellular uptake of nucleic acids using receptors that recognize an amino acid sequence. It has been reported that when an amino acid sequence recognized by receptors has been chemically introduced into a water-soluble polymer, liposome, granule, and polymer micelle, and the like, the intracellular uptake of the nucleic acid via the receptors that recognize the amino acid sequence in the carrier was enhanced. Moreover, it is known that a substance comprising the amino acid sequence and polymerized interacts efficiently with cell surface receptors.

On the other hand, with respect to receptors that recognize sugar molecules, it has been reported that by using a nucleic acid carrier wherein monosaccharide molecule or disaccharide molecule is chemically bonded to a water-soluble polymer, the nucleic acid uptake is increased through uptake of the carrier via sugar recognizing receptors. However, at present there have been no reports that a polysaccharide comprising monosaccharide molecules, disaccharide molecules, or trisaccharide molecules themselves are linked together and polymerized has increased the intracellular uptake of a nucleic acid. There are many types of sugar recognizing receptors present on the cell surface depending on the types of sugar molecules to be recognized. For example, because glucose is an essential substance for the survival of the cell, most cells have receptors that recognize glucose on the cell surface for more efficient uptake of glucose. It is known that receptors that recognize galactose or mannose and the like are present on immunocompetent cells such as macrophages, monocytes, lymphocytes, and dendrocytes, and receptors that recognize galactose are present on bone marrow cells. Even though it is known that these sugar recognizing receptors are present on the cell surface, there have been no reports concerning the intracellular uptake of a nucleic acid via these receptors and a polysaccharide. Because the type of the sugar receptor is different depending on the cell type, the uptake of a nucleic acid by specific cells becomes possible by utilizing this difference. For example, parenchymal cells of the liver have galactose recognizing receptors on the cell surface. It has been reported that pullulan, a polysaccharide recognized by the galactose recognizing receptors, has capability to target a nucleic acid to the liver in the body. By using its property of interacting with galactose residue recognizing receptors, the specific accumulation (targeting) of intravenously administered pullulan in the liver has been reported. The reason for an increased biological activity of a nucleic acid in the liver was targeting a nucleic acid complexed with pullulan to the liver. The report does not state that intracellular uptake of the nucleic acid was increased by pullulan.

It is known that stem cells, for example, hematopoietic stem cells and undifferentiated mesenchymal stem cells that can be collected from the bone marrow and tissue stem cells that can be collected from tissues and organs such as fat, skin, muscle, nerve, liver and pancreas, differ from somatic cells in that they have lower nucleic acid uptake efficiency. In the prior art nucleic acid carriers based on a cationized polymer or a cationized liposome have passed through the research and development phase and have appeared on the market. Unlike the case with common cells, however, almost no acceptable results have been obtained by attempts to transfect a stem cell with a nucleic acid using these carriers. The reason for the difference in uptake efficiency between common cells and stem cells is not understood at the present time. In addition, stem cells weaken and die more easily than common cells when cultured together with a cationized carrier. It is believed that this phenomenon occurs because negative charges on the cell surface and positive charges on the nucleic acid-carrier complex interact, which interferes with the fluidity of the cell membrane and leads to cell death. In contrast, uptake based on interaction between a cell and a nucleic acid-carrier via receptors has little effect on the fluidity of the cell membrane and, as a result, the cytotoxicity decreases. Attempts have been made to increase the intracellular uptake of a nucleic acid by temporarily elevating the permeability of the cell membrane through the application of electrical pulses, ultrasonic irradiation, and the like, but there is a problem because physical stimulation of this nature is very damaging to the cells, and the cell survival rate is low.

The following documents are prior art documents related to the present invention: WO 01/34206 and JP 2003-104914.

### DISCLOSURE OF THE INVENTION

An object of the present invention is to provide a novel pharmaceutical preparation for efficiently transfecting a cell with a nucleic acid.

The inventors have discovered that the efficiency of nucleic acid uptake into cells is increased by transfecting the cells with a nucleic acid using a polymer wherein a sugar molecule recognized by a sugar recognizing receptor, i.e., a polysaccharide is attached to itself,.

The present invention provides a composition comprising a polysaccharide for the purpose of promoting transfection of a cell with a nucleic acid. The present invention also provides a method for transfecting a cell with a nucleic acid wherein a polyionic complex of the nucleic acid and polysaccharide is formed, and the polyionic complex is taken up by the aforementioned cells. Preferably, the cells are selected from bone marrow mesenchymal stem cells, nervous system cell lines, stem cells from adipose tissue, immune cells, neurons, chondrocytes, epithelial cells, primary culture cells, and cancer cells. Also preferably, the polysaccharide is a cationized pullulan derivative, a cationized dextran derivative, or a cationized mannan derivative.

### BRIEF DESCRIPTION OF THE DRAWINGS

Figure 1 shows luciferase activity after transfection of undifferentiated mesenchymal stem cells with the luciferase gene;
Figure 2 shows luciferase activity after transfection of a cell line derived from neurons with the luciferase gene;
Figure 3 shows luciferase activity after transfection of human adipose tissue stem cells with the luciferase gene;
Figure 4 is a result of analysis by FACS of the expression efficiency of the EGFP gene introduced into human dendrocytes;
Figure 5 shows luciferase activity after transfection of murine bone marrow stem cells (MSC) with the luciferase gene;
Figure 6 shows luciferase activity with varied amounts of the introduced luciferase gene;
Figure 7 shows luciferase activity when the cells are transfected with a luciferase gene using pullulans of different molecular weights;
Figure 8 shows luciferase activity of the cells transfected with the luciferase gene when the spermine introduction rate in pullulans of different molecular weights is varied;
Figure 9 shows luciferase activity after transfection of cancer cells with the luciferase gene;
Figure 10 shows luciferase activity after transfection of cancer cells with the luciferase gene;
Figure 11 shows luciferase activity after transfection of cancer cells with the luciferase gene;
Figure 12 shows luciferase activity after transfection of stem cells with the luciferase gene;
Figure 13 shows luciferase activity after transfection of primary culture cells with the luciferase gene;
Figure 14 shows luciferase activity after transfection of alveolar epithelium type II RLE6TN cells with the luciferase gene;
Figure 15 shows luciferase activity after transfection of mouse peritoneal macrophages with the luciferase gene;
Figure 16 shows luciferase activity after transfection of rat peritoneal macrophages with the luciferase gene; and
Figure 17 shows luciferase activity after transfection of mouse ES cells with the luciferase gene.

### PREFERRED EMBODIMENTS OF THE INVENTION

When sugar recognizing receptors are roughly classified, three types that recognize glucose, mannose, and galactose, are particularly well known. For example, amylose, amylopectin, or dextran, glucomannan, and pullulan, which are polymers wherein the respective sugars are linked together, as well as various derivatives and the like thereof are exemplified as a polysaccharide that binds to these receptors. Furthermore, provided the polysaccharide is recognized by a sugar recognizing receptor, selection of the polysaccharide does not depend on the physicochemical properties of the polysaccharide, such as solubility (i.e., solubility or insolubility in water), branching, and the like. In addition, regardless of whether the polysaccharide is originally from an animal, plant, microorganism, etc., or whether it is a recombinant product, it can be used as the nucleic acid carrier in the present invention. In one method for attachment of these polysaccharides to the nucleic acid, the polysaccharide can be cationized, for example, by a chemical reaction between a the polysaccharide and a diamine compound to introduce an amino group. Any of the publicly known reaction technology can be used for affecting this attachment reaction. More specifically, a primary, secondary, tertiary, or quaternary amino group can be chemically introduced such that it forms a polyionic complex with the negative charges of the nucleic acid. To effect intracellular uptake via the hyaluronic acid receptor (CD44) or the acetylglucosamine receptor, it is possible to use hyaluronic acid, chitin, or chitosan and the like as the nucleic acid carrier. A complex or a mixture of a plurality of the aforementioned polysaccharides or derivatives thereof can be used as the polysaccharide carrier. It is possible to check whether the polysaccharide-nucleic acid polyionic complex has been taken up by the cell via the sugar recognizing receptors by evaluating whether the uptake of the nucleic acid decreases or not after treating the cells with a recognized monosaccharide or substance, and blocking the sugar recognizing receptors beforehand.

In the present invention, any type of polysaccharide can be used. The polysaccharide is not particularly limited to the following description, but one example of a polysaccharide is pullulan, which is a water-soluble polysaccharide. Pullulan is an α-glucan produced by fermentation of a partial hydrolysate of starch by Aureobasidium pullulans. It is a straight-chain, water-soluble polymer wherein maltotriose, which consists of 3 glucose moieties, is linked by α-1,6 glucosidic linkages. It is known that the intracellular uptake of pullulan is via the asialo-glycoprotein receptor expressed on the cell surface. Pullulan products of various molecular weights are commercially available, but the molecular weight of pullulan used in the present invention is preferably approximately 2,000 to 500,000, and more preferably approximately 20,000 to 100,000. Even if a different species of polysaccharide is used, the preferred molecular weight lies within the aforementioned range.

Especially preferable polysaccharides for forming the polyionic complex with the nucleic acid in the present invention are a cationized pullulan derivative wherein a cationic group is introduced into pullulan, a cationized dextran derivative wherein a cationic group is introduced into dextran, and cationized mannan wherein a cationic group is introduced into mannan. The process of cationization is not particularly limited provided the functional group to be cationized can be introduced under physiological conditions. In a preferred method, primary, secondary, or tertiary amino groups or ammonium groups are added to the hydroxyl groups on the pullulan molecule under a mild conditions. For example, an alkyl diamine such as ethylenediamine, N,N-dimethyl-1,3-diaminopropane, trimethyl ammonium acetohydrazide, spermine, spermidine, or diethylamide chloride can be reacted using various condensation reagents such as 1-ethyl-3-(3-dimethylaminopropyl) carbodiimide hydrochloride, cyanuric chloride, N,N'-carbo diimidazole, cyanogen bromide, a diepoxy compound, tosyl chloride, a dianhydride compound such as diethyl triamine-N,N,N'N " ,N " -pentanoic acid dianhydride, trisyl chloride and the like. In particular, the method wherein ethylene diamine or spermine is reacted is most suitable because it is simple and widely applicable. The introduction rate of the cationic group used in the present invention is preferably 2% to 90%, more preferably 5% to 60%, and most preferably 10% to 30% when expressed as a ratio (mole % ratio) of introduced spermine with respect to the hydroxyl groups present on the polysaccharide. Even if a different species of polysaccharide is used, the preferred mole % ratio lies within the aforementioned range.

The nucleic acid used in the present invention includes DNA, RNA, dsRNA, an DNA-RNA complex, PNA and derivatives thereof having a modification in the saccharide, phosphate or base. Examples of the nucleic acid include a gene that encodes a protein useful for the treatment of various diseases, a vector containing the same, antisense DNA, siRNA, and decoy DNA. Examples of a gene that encodes a protein necessary for the treatment of a disease include a gene for a low molecular weight peptide such as a hormone; or interferon, interleukin, cytokine, chemokine, cell growth factor; or matrix metalloprotease (MMP) and the like; a partial peptide containing the physiologically active site of these proteins; or a neutralizing antibody or receptor agonist and the like of these proteins and peptides. Examples of low molecular weight peptide such as a hormone include substances such as interferon, although the low molecular weight peptides are not particularly limited to the same provided they are suitable for treatment. Examples of cell growth factors include hGH EGF, NGH, HGF, FGF, HB-EGF, IGF and the like and fragments thereof, for example, NK4, which is an intramolecular fragment of HGF.

DNA that encodes a protein can be obtained by cloning from a genomic or cDNA library based on a known sequence, or it may be produced by chemical synthesis. The DNA that encodes the protein is inserted into a plasmid-based vector so that the functional protein can be expressed in the cell. The plasmid-based vector includes a promoter region, start codon, stop codon, and terminator region arranged in a manner such that the DNA will be transcribed in the cell and the protein encoded thereby is suitably expressed. Such a plasmid-based vector can be easily prepared by inserting the desired DNA into an expression vector available in the art via a suitable restriction enzyme site. It is also possible to prepare the vector by means of synthesis or partial synthesis based on the base sequence of the DNA to be inserted. The type of promoter, start codon, stop codon, and terminator region in the plasmid-based vector are not particularly limited.

Provided it has the desired activity, the protein to be expressed from the nucleic acid used in the present invention may have one or more amino acids in its amino acid sequence substituted, deleted, or added, and similarly, a sugar chain may be substituted, deleted, or added thereto. In addition, the protein may be produced as a fusion protein together with another protein or fragment thereof. Therefore, the DNA may encode a protein of such a modified type. Methods of preparing DNA that encodes such a modified protein by site-directed mutation, gene recombination, or synthesis are well known in the art.

To achieve uptake of the nucleic acid into the cell with the aid of the polysaccharide, it is necessary to prepare a complex of the nucleic acid and the polysaccharide. The method for producing of this complex is not particularly limited provided the complex has the property of interacting with sugar recognizing receptors on the cell. For example, the complex of the polysaccharide and the nucleic acid can be obtained by using a polyionic complex formed by the interaction of positive charges and negative charges, or by utilizing physicochemical interactions such as hydrogen bonds, hydrophobic interactions, coordinate bonds and the like.

The polyionic complex is a complex formed by the binding of the nucleic acid and the water-soluble polysaccharide via an ionic bond. The polyionic complex comprising the nucleic acid and a cationized pullulan derivative, a cationized dextran derivative or cationized mannan derivative can be formed by mixing the nucleic acid and the cationized pullulan derivative, cationized dextran derivative or cationized mannan derivative in a suitable buffer solution and letting it stand for a specified time. The reaction can be performed at room temperature. The formation of the polyionic complex can be monitored with the turbidity of the mixture as an indicator. The properties of the polyionic complex can be adjusted by selecting the molecular weight of the pullulan to be used and the extent of cationization. The extent of the cationization can be measured using the ratio of the number of moles of amino groups in the cationized pullulan derivative, cationized dextran derivative or cationized mannan derivative and the number of moles of phosphate groups in the nucleic acid as an indicator. The optimal molecular weight and the extent of cationization for uptake into the cells can be selected based on the type of cells and nucleic acid to be used. By forming the polyionic complex between the nucleic acid and the cationized pullulan derivative, cationized dextran derivative or cationized mannan derivative according to the present invention, the negative charge of the nucleic acid is neutralized, and a decrease in molecular size is caused by the weakening of electrical repulsion. Moreover, the nucleic acid is stabilized within a pharmaceutical preparation. In addition, the nucleic acid can be taken into the cell with high efficiency due to the strong affinity between the pullulan and the asialo-glycoprotein receptor on the cell surface.

To effect transfection of a cell with the polyionic complex of the nucleic acid and the cationized pullulan derivative, cationized dextran derivative or cationized mannan derivative, the cell is cultured with a suitable medium and then the polyionic complex is added to the medium and cultured at 37°C for 2 hours to 2 days more. The amount and concentration of the polyionic complex to be added and the incubation conditions can be properly selected in accordance with the properties of the cells to be used.

The composition of the present invention can be used not only in an in vitro culture system, but also in vivo to transfect cells in the body of an organism with the nucleic acid. For example, the solution of the polyionic complex of the nucleic acid and cationized pullulan derivative, cationized dextran derivative or cationized mannan derivative can be administered to the muscle of an animal's foot near the nerves. One to 2 days later, the expression of biological activity due to the transfection of the proximal neurons surrounding the injection site with the nucleic acid can then be measured.

By administering an aqueous polyionic complex solution into the body, cells in the tissue surrounding the injection site can be transfected with the nucleic acid. The present invention does not limit the site of administration, and examples of administration include subcutaneous, intracutaneous, intravascular, intramuscular, intrathoracic, intracardial, intraperitoneal, intramedullary, intrabronchial, intranasal, intracerebral, intratumoral, intestinal, intravaginal, intravesical, intra-urinary tract, intralymphatic, intravitreal, subretinal, and the like. The present invention is not limited to the dosage form, and examples include dosage forms wherein particles, solids, rods, granules, films and the like of the present invention are mixed or complexed with a carrier to form an aqueous solution or dispersion.

The entire content of the patents and reference documents specifically cited in Description are incorporated herein by reference. In addition the entire content of the Description and Drawings Japanese Patent Application 2004-103568, which forms the basis for the priority claim of this application, are incorporated herein by reference.

The present invention is explained in greater detail below through the Examples, but the present invention is by no means limited to these examples.

### EXAMPLE 1

### Preparation of cationized pullulan derivative and cationized gelatin

The manufacture of cationized pullulan derivative was performed by the reaction of N,N'-bis (3-aminopropyl)-1,4-butanediamine (spermine) with the hydroxyl groups of pullulan. The pullulan (weight average molecular weight 48,000 or 100,000, Showa Denko) was dissolved in anhydrous dimethyl sulfoxide (10 mg/ml). Next, 0.252 M of N,N'-carbonyl diimidazole (CDI, Nacalai Tesque) and 2.52 M of spermine (Sigma) were added, and the mixture was stirred for 20 hours at room temperature. The reaction solution was dialyzed against distilled water for 2 days, and the dialysate was lyophilized to obtain the spermine-introduced cationized pullulan derivative (spermine-pullulan).

Cationized gelatin was prepared by the chemical reaction of an amine compound such as ethylenediamine, spermidine, spermine and the like with the carboxyl groups of the gelatin (Mw=100,000, isoelectric point = 9.0, pig skin collagen, acid treated, Nitta Gelatin). Specifically, 250 ml of 0.1 M phosphate buffer (pH=5) was added to 10 g of gelatin, and stirred for 1 hour at 40°C to obtain a gelatin solution. Fifty mole equivalents of the amine compound with respect to the carboxyl groups of the gelatin was added, and after concentrated hydrochloric acid (Nacalai Tesque) was added to the aqueous solution to adjust the pH to 5, 5.35 g of 1-ethyl-3-(3-dimethylaminopropyl) carbodiimide hydrochloride (EDC, Nacalai Tesque) (3 mole equivalents with respect to the carboxyl groups of the gelatin) was added. After 0.1 M phosphate buffer was added to adjust the total volume to 500 mL (final gelatin concentration: 0.02 g/mL), the mixture was reacted for 18 hours at 37°C with stirring. After the reaction was completed, the mixture was dialyzed against distilled water for 2 days, and the dialysate was lyophilized to obtain the cationized gelatin (the notation of the cationized gelatin was based on the introduced amine compound and expressed as E50, SD50 and SM50 representing ethylenediamine, spermidine, and spermine, respectively).

### EXAMPLE 2

### Preparation of plasmid DNA

Plasmid DNA containing the gene encoding luciferase (pCMV-luc) was used as plasmid DNA. After E. coli were transformed with pCMV-luc containing an ampicillin resistance gene, they were cultured for 18 hours at 37°C in LB medium containing ampicillin. The E. coli that grew were recovered by centrifugation, and the plasmid DNA was extracted by alkaline-SDS method. To evaluate the purity of the plasmid DNA, the ratio of the absorption at 260nm/280nm of the obtained plasmid DNA solution was measured and found to be 1.8-2.0.

### EXAMPLE 3

### Measurement of amino group introduction rate

The spermine introduction rate in the spermine-introduced cationized pullulan derivative was calculated by quantitation of the amino groups using the 2,4,6-trinitrobenzene sulfonic acid (TNBS) method. More specifically, 100 µL of 0.2 M phosphate buffer saline (PBS, pH 7.4), 200 µL of 4wt% sodium bicarbonate solution, and 200 µL of 0.1wt% TNBS solution were added to 100µL of spermine-pullulan solution and reacted for 2 hours at 37°C. Then absorbance of this reaction solution at 415 nm was measured. When the spermine introduction rate per hydroxyl group was calculated in pullulan having a molecular weight of 48,000 and 100,000 from a calibration curve using this absorbance value and β-alanine, the rates were 28.9 ± 0.08% and 32.3 ± 0.18%, respectively.

The amine introduction rate of the cationized gelatin was calculated in a similar manner by the TNBS method. When the introduction rate of the amine compound with respect to the carboxyl groups of the gelatin was calculated for E50, SD50, and SM50, it was 47.8%, 45.9%, and 49.0%, respectively.

### EXAMPLE 4

### Polyionic complex formation

The polyionic complex consisting of the cationized pullulan derivative and the plasmid DNA was formed by mixing equal volumes (50 µL) of spermine-pullulan aqueous solution (molecular weight 48, 000: 0.21 mg/mL, molecular weight 100,000: 0.20 mg/mL) and PBS containing pCMV-luc (100 µg/ml), and letting the mixture stand for 15 minutes at room temperature.

The polyionic complex consisting of a cationized gelatin and plasmid DNA was formed by mixing equal volumes (25 µL) of cationized gelatin aqueous solution (E50 (0.92 mg/mL), SD50 (1.18 mg/mL), SM50 (0.85 mg/mL), and PBS containing pCMV-luc (200 µg/mL), and letting the mixture stand for 15 minutes at room temperature.

The polyionic complex consisting of LipofectAmine™ (registered trade-mark) and plasmid DNA was formed by mixing equal volumes (100 µL) of LipofectAmine™ (0.1 mg/mL) and PBS containing pCMV-luc (50 µg/mL), and letting the mixture stand for 15 minutes at room temperature.

### EXAMPLE 5

### Transfection of undifferentiated mesenchymal stem cells with DNA

Isolated undifferentiated mesenchymal stem cells (passage number 2) were used as the recipient cells. Bone marrow from femurs and tibias of F344 rats (3-week-old males) was rinsed in 1 mL of PBS, and was placed in T25 culture flasks (Corning) containing 5mL of MEM Alpha (αMEM-FCS, Gibco) with 15 vol% calf fetal serum (FCS). The medium was replace every 3 days, and subcultured once the cells grew confluent. Thereafter, the medium was replaced every 3 days, and cells that had become confluent were used in the experiment. The cells were inoculated into six-well plates (Costar) at a concentration of 1×10⁴ cells/cm2 (1×10⁵ cells/well) and cultured with αMEM-FCS in 5% CO₂-95% air at 37°C for 24 hours. After replacing the medium with serum-free ocMEM medium, the polyionic complex consisting of pCMV-luc (100 µL) and either spermine-pullulan, cationized gelatin, or LipofectAmine™ (100 µL, 50 µL, and 200 µL, respectively) was added, and culturing was continued for additional 6 hours. Next, the medium was replaced with αMEM-FCS and culturing was continued for 24 hours. After the medium was removed and the cells were thoroughly rinsed with PBS, a cytolytic solution (25 mM Tris-phosphate buffer (pH7.8), 2 mM dithiothreitol, 2 mM 1,2-diaminocyclohexane-N,N,N',N'-tetraacetic acid, 10% glycerol, and 1% Triton™ X-100) was added to lyse the cells. The gene expression level was quantitated based on measurement of the chemiluminescence from the luciferase protein in the cell lysate. The amount of total protein in the cell lysate was measured by the Bicinchoninate (BCA) method.

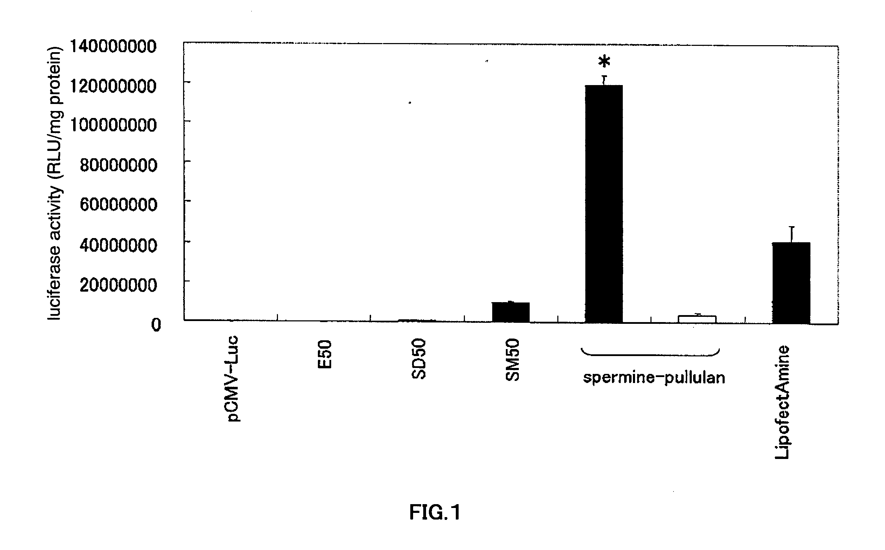

Figure 1 shows the results of transfection of undifferentiated mesenchymal stem cells with the luciferase gene. In the figure, the black bar represents no addition of asialofetuin, the white bar represents addition of asialofetuin, and * indicates p<0.05. The results show that the gene expression was enhanced in the complex consisting of pCMV-luc and cationized gelatin and the complex consisting of pCMV-luc and LipofectAmine™, and that the gene expression was enhanced even more by the complex consisting of pCMV-luc and spermine-pullulan.

The expression level decreased significantly when asialofetuin (1 mg/mL), which binds to the asialo-glycoprotein receptor, was added concurrently with the polyionic complex. Since the asialo-glycoprotein receptor is known to be present in bone marrow tissue, this result indicates that the polyionic complex of spermine-pullulan and plasmid DNA is taken up by undifferentiated mesenchymal stem cells via the asialo-glycoprotein receptor. Gene expression was detected at extremely low levels with the cationized gelatins E50, SD50, and SM50.

### EXAMPLE 6

### Transfection of nervous system cell line with DNA

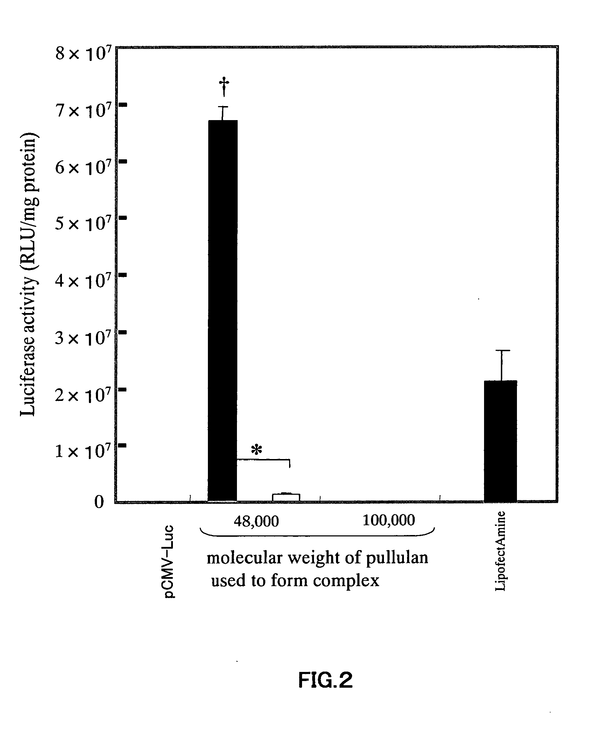

PC 12 cells were inoculated into six-well plates (Costar) at a concentration of 4×10⁴ cells/cm² (4×10⁵ cells/well) and cultured in MEM-FCS with 10 vol% fetal calf serum (FCS) in 5% CO₂-95% air at 37°C for 24 hours. After replacing the medium with serum-free Opti-MEM (Gibco) medium, the polyionic complex (DNA content: 5 µg/well) consisting of pCMV-luc (100 µL) and either spermine-pullulan or LipofectAmine™. (100 µL and 200 µL, respectively) was added, and culturing was continued for additional 6 hours. Next, the medium was replaced with MEM-FCS and culturing was continued for 42 hours. After the medium was removed and the cells were thoroughly rinsed with PBS, a cytolytic solution (25 mM Tris-phosphate buffer (pH7.8), 2 mM dithiothreitol, 2 mM 1,2-diaminocyclohexane-N,N,N',N'-tetraacetic acid, 10% glycerol, and 1% Triton™ X-100) was added to lyse the cells. The gene expression level was quantitated based on measurement of the chemiluminescence of the luciferase protein in the cell lysate. The amount of total protein in the cell lysate was measured by the Bicinchoninate (BCA) method. Figure 2 shows the results. In the figure, the black bar represents no addition of asialofetuin, the white bar represents addition of asialofetuin, and * indicates p<0.05. The dagger (t) indicates p<0.05 with respect to the molecular weight of 100,000 and the LipofectAmine™.

### EXAMPLE 7

### Transfection of human adipose tissue stem cells with DNA

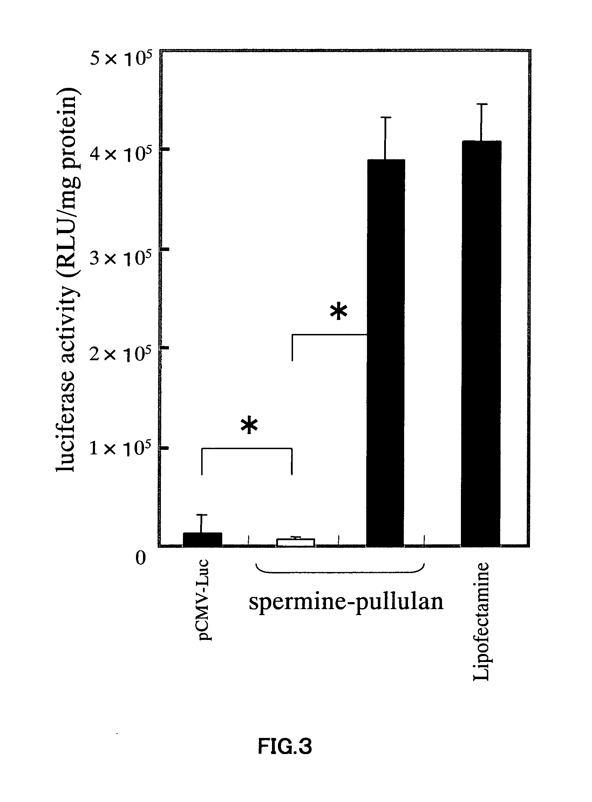

Human adipose tissue stem cells (passage number 2) were used as the recipient cells. Under the informed consent from patients, human adipose tissue (5 mL) was collected during excision of breast cancer and cut up with scissors, and the tissue was digested by treatment in an aqueous solution of 2 mg/mL of collagenase S1 (provided by Nitta Gelatin Co., Ltd.) at 37°C for 15 to 20 minutes. The digested tissue was filtered through a nylon mesh (pore size: 200 pm) and subjected to centrifugation (4°C, 1,000 rpm, 5 minutes) to obtain the cellular component containing human adipose tissue stem cells. After rinsing the cells twice with Medium199 with 15 vol% FCS (Medium199-FCS), the obtained cells were inoculated into T25 culture flasks. The cells were cultured in Medium199-FCS containing bFGF (0.1 µg/mL). The medium was replaced every 3 days, and subcultured once the cells grew confluent. Thereafter, the medium was replaced every 3 days, and the cells that had become confluent were used in the experiment. The cells were inoculated into six-well plates at a concentration of 1×10⁴ cells/cm² (1×10⁵ cells/well) and cultured with Medium199-FCS in 5% CO₂-95% air at 37°C for 24 hours. After replacing the medium with serum-free Medium199 Medium, the polyionic complex (DNA content: 5 µg/well) consisting of pCMV-luc (100 µL) and either spermine-pullulan or LipofectAmine™. (100 µL and 200 µL, respectively) was added, and culturing was continued for additional 6 hours. Next, the medium was replaced with Medium199-FCS and culturing was continued for 24 hours. After the medium was removed and the cells were rinsed thoroughly with PBS, the cytolytic solution was added to lyse the cells. The gene expression level was quantitated based on measurement of the chemiluminescence from the luciferase protein in the cell lysate. The amount of total protein in the cell lysate was measured by the BCA method. Figure 3 shows the results. In the figure, the black bar represents no addition of asialofetuin, the white bar represents addition of asialofetuin, and * indicates p<0.05.

### EXAMPLE 8

### Transfection of human dendrocytes with DNA

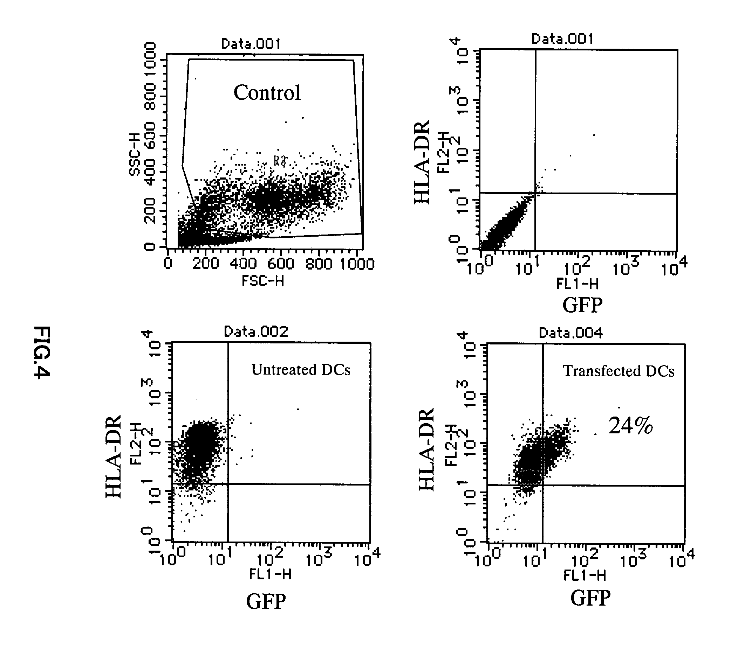

Human dendrocytes were used as the recipient cells. More specifically, heparin-treated peripheral blood was collected from healthy humans, diluted two-fold in PBS, and gently separated and layered on 15 mL of Ficoll-Paque solution. After centrifugation for 15 minutes at 800 g, the intermediate layer (monocytes) was collected with a sterile pipette, rinsed with RPMI1640, centrifuged again, and the cells were collected. The monocytes (1×10⁷ cells) were suspended in 10 mL of RPMI1640 medium with 10% FCS (RPMI1640-FCS) in T25 culturing flasks. After 2 hours or more, the supernatant containing floating cells was aspirated off and discarded, 10 mL of RPMI1640-FCS with 50 ng/mL of rhIL-4 and 50 ng/mL of rhGM-CSF was added to the adhere cells, which were then culture for 5 to 7 days and used in the experiment. Supernatant of the flasks cultured for 5 to 7 days was collected by aspiration and centrifuged to obtain undifferentiated human dendrocytes. The human dendrocytes in RPMI1640 were inoculated into six-well plates at a concentration of 3 to 5×10⁵ cells/well, polyionic complex (100 µL) (DNA content: 5 µg/well) consisting of 200 µg/mL pEGPF (50 µL) and 0.4 mg/mL spermine-pullulan (50 µL) was added, and the plates were centrifuged for 10 minutes at 200xg. The RPMI1640-FCS was replaced after 2 to 6 hours. EGFP expression was analyzed by flow cytometry (FACS). More specifically, the cells were collected by aspiration 24 to 48 hours after transfection, rinsed once with PBS, and HLA-DR labeled with PE and CD80 antibody were added. The cells were let stand in the dark for 30 minutes at 4°C, and rinsed again with PBS and analyzed. GFP was detected with FL1 and PE was detected with FL2. Figure 4 shows the results. When pEGFP and spermine-pullulan were used as the polyionic complex, the expression efficiency was 24-30%, while it was 0% with LipofectAmine™. Almost no cell death occurred with spermine-pullulan, whereas almost all the cells died with the LipofectAmine™ complex.

### EXAMPLE 9

### Transfection of chondrocytes with DNA

Chondrocytes collected from rabbit joint cartilage were transfected with pCMV-luc (100 µL) in the same manner as in Example 5 and the gene expression was tested. The expression level was higher with spermine-pullulan than with LipofectAmine™.

### EXAMPLE 10

### Transfection of skin epithelial cells with DNA

Cultured skin epithelial cells were transfected with pCMV-luc (100 µL) in the same manner as in Example 5 and the gene expression was tested. The expression level was higher with spermine-pullulan than with LipofectAmine™.

### EXAMPLE 11

### Transfection of corneal epithelial cells with DNA

Cultured corneal epithelial cells were transfected with pCMV-luc (100 µL) in the same manner as in Example 5 and the gene expression was tested. The expression level was higher with spermine-pullulan than with LipofectAmine™.

### EXAMPLE 12

### Transfection of pigmented epithelial cells with DNA

Cultured pigmented epithelial cells were transfected with pCMV-luc (100 µL) in the same manner as in Example 5 and the gene expression was tested. The expression level was higher with spermine-pullulan than with LipofectAmine™.

### EXAMPLE 13

### Transfection of tubule epithelial cells with DNA

Cultured tubule epithelial cells were transfected with pCMV-luc (100 µL) in the same manner as in Example 5 and the gene expression was tested. The expression level was higher with spermine-pullulan than with LipofectAmine™.

### EXAMPLE 14

### Transfection of cochlear hair cells with DNA

Cultured cochlear hair cells were transfected with pCMV-luc (100 µL) in the same manner as in Example 5 and the gene expression was tested. The expression level was higher with spermine-pullulan than with LipofectAmine™.

### EXAMPLE 15

### Transfection of keratinocytes with DNA

Cultured keratinocytes were transfected with pCMV-luc (100 µL) in the same manner as in Example 5 and the gene expression was tested. The expression level was higher with spermine-pullulan than with LipofectAmine™.

### EXAMPLE 16

### Transfection of mesangial cell with DNA

Cultured mesangial cells were transfected with pCMV-luc (100 µL) in the same manner as in Example 5 and the gene expression was tested. The expression level was higher with spermine-pullulan than with LipofectAmine™.

### EXAMPLE 17

### Transfection of extra-glomerular mesangial cells with DNA

Cultured extra-glomerular mesangial cells were transfected with pCMV-luc (100 µL) in the same manner as in Example 5 and the gene expression was tested. The expression level was higher with spermine-pullulan than with LipofectAmine™.

### EXAMPLE 18

### Transfection of hair papilla cells with DNA

Cultured hair papilla cells were transfected with pCMV-luc (100 µL) in the same manner as in Example 5 and the gene expression was tested. The expression level was higher with spermine-pullulan than with LipofectAmine™.

### EXAMPLE 19

### Transfection of hair matrix basal cells with DNA

Cultured hair matrix basal cells were transfected with pCMV-luc (100 µL) in the same manner as in Example 5 and the gene expression was tested. The expression level was higher with spermine-pullulan than with LipofectAmine™.

### EXAMPLE 20

### Transfection of hair follicle basal cells with DNA

Cultured hair follicle basal cells were transfected wit pCMV-luc (100 µL) in the same manner as in Example 5 and the gene expression was tested. The expression level was higher with spermine-pullulan than with LipofectAmine™.

### EXAMPLE 21

### Transfection of melanoma cancer cells with DNA

Cultured melanoma cancer cells were transfected with pCMV-luc (100 µL) in the same manner as in Example 5 and the gene expression was tested. The expression level was higher with spermine-pullulan than with LipofectAmine™.

### EXAMPLE 22

### Transfection of nerve cells with DNA in vivo

An aqueous solution of polyionic complex consisting of a cationized pullulan derivative and a nucleic acid was injected into the femoral muscles of mice. After 2 days, intramuscular nerve cells and the proximal nerve cells connected thereto were collected, and the gene expression was tested. The level of expression was significantly higher with spermine-pullulan than with LipofectAmine™.

### EXAMPLE 23

### Transfection of murine bone marrow stem cells with DNA

Aqueous solutions of polyionic complexes consisting of a cationized polysaccharide (spermine-pullulan, spermine-dextran, spermine-mannan, and spermine-amylopectin) and a nucleic acid were prepared. Spermine was introduced to pullulan, dextran, mannan, and amylopectin in the same manner as in Example 1. The spermine introduction rates were as follows: pullulan (12.3%), dextran (9.51%), mannan (13.3%), and amylopectin (12.3%). Next, cationized polysaccharide solutions with the concentrations shown in the following table were prepared.

**Table 1**

| Concentration of cationized polysaccharide (µg/mL) | | | |
|---|---|---|---|
| N/P ratio | 1 | 3 | 5 |
| Spermine-introduced pullulan (MW: 47300) | 71.3 | 214 | 357 |
| Spermine-introduced dextran | 85.1 | 255 | 425 |
| Spermine-introduced mannan | 67.8 | 203 | 339 |
| Spermine-introduced amylopectin | 71.3 | 214 | 357 |

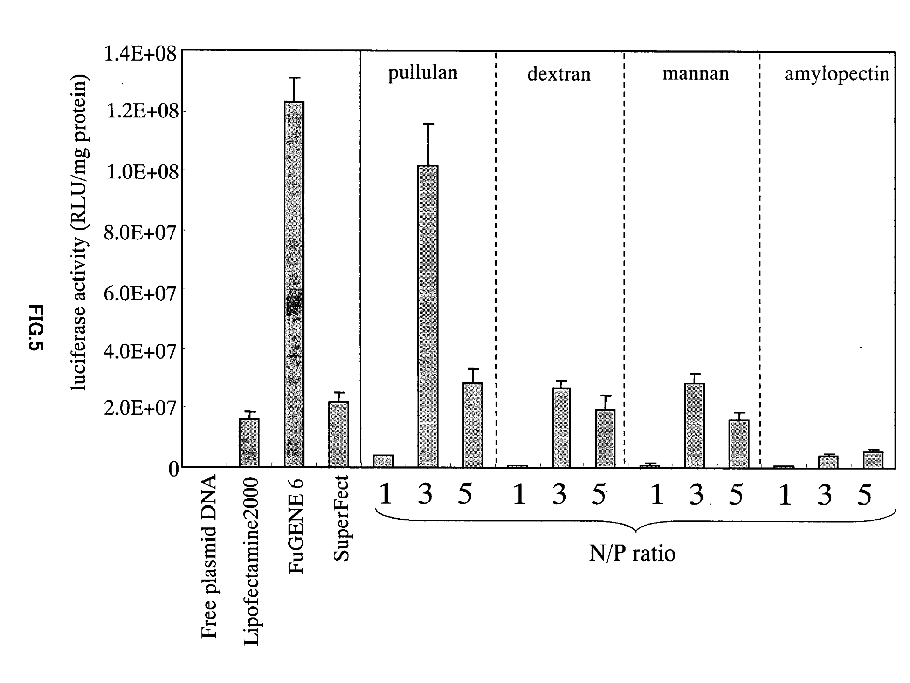

This aqueous solution was mixed with an equal volume of plasmid solution at a concentration of 100 µg/mL and let stand at room temperature for 15 minutes. The indicated amount of each polyionic complex was added to the culture of murine bone marrow stem cells (MSC), and the cells were cultured for 6 hours in serum-free medium. Next, the cells were transfected with the polyionic complex by incubation with the complex for 24 hours in a medium containing serum, and the gene expression was tested (Figure 5). When the spermine-pullulan (N/P ratio 3) was used, the expression level was roughly the same as with FuGENE6™, but was significantly higher than with LipofectAmine2000™ and SuperFect™. Moreover, the expression level was significantly higher with spermine-dextran and spermine-mannan at the N/P ratio of 3 compared with LipofectAmine2000™.

### EXAMPLE 24

### Change in transfection efficiency according to quantity of DNA

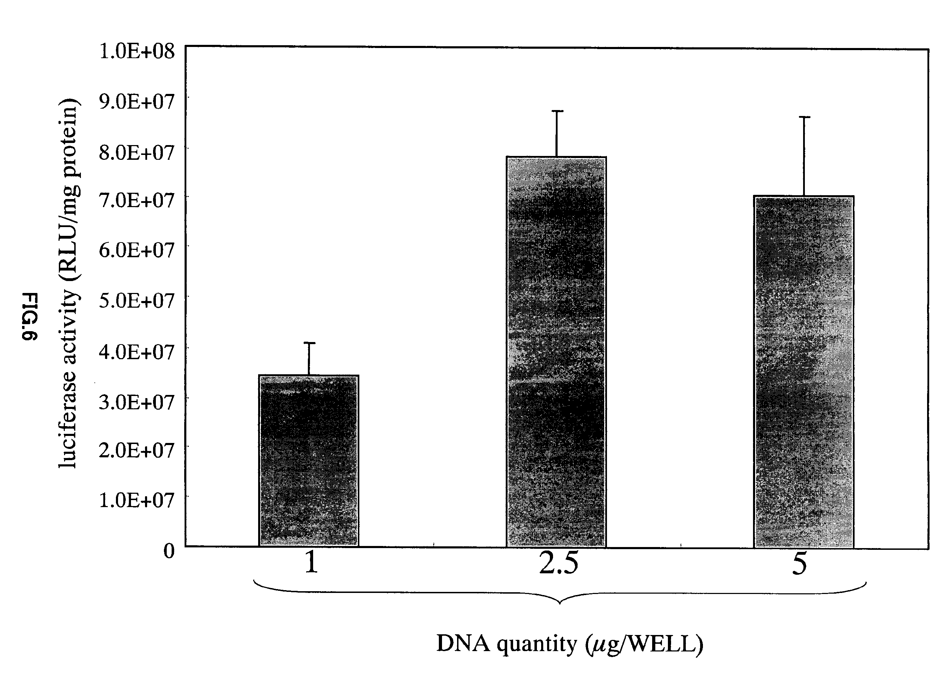

The cells were transfected with the plasmid using spermine-pullulan (pullulan molecular weight 47300, CDI 3.0, N/P ratio 3, spermine introduction rate: 20.4%) in the same manner as in Example 23 with varying the amount of pCMV-luc (100 µL), and the gene expression was tested. The expression level was highest at a DNA quantity of 2.5 µg/well (Figure 6).

### EXAMPLE 25

### Change in transfection efficiency with pullulans of different molecular weights

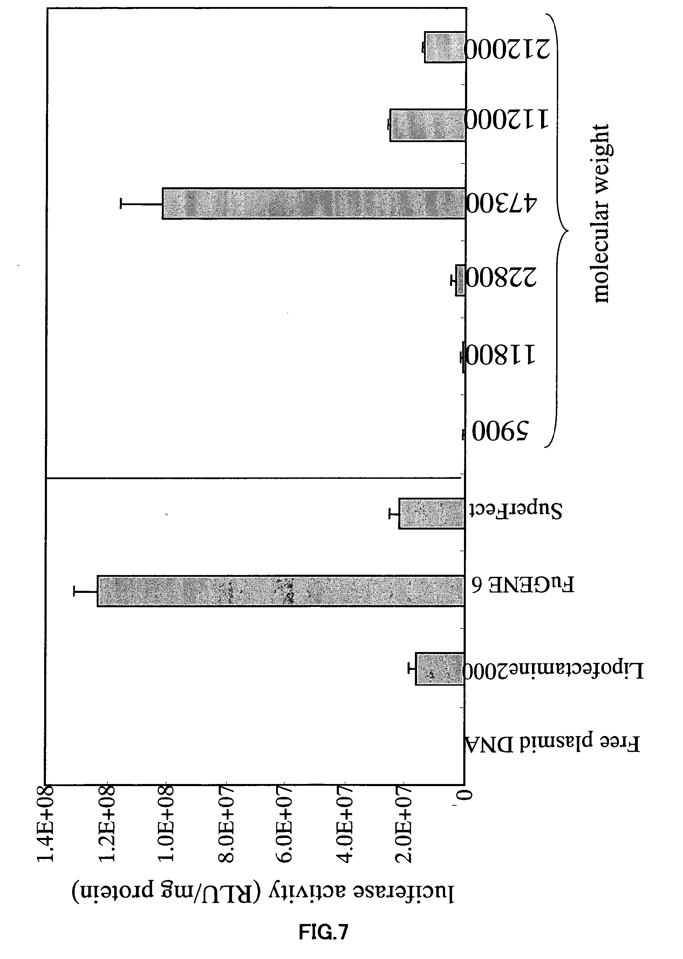

The cells were transfected with pCMV-luc (100 µL) using spermine-pullulan (pullulan molecular weights from 5900 to 212000, CDI 1.5, N/P ratio 3, spermine introduction rate: 5900;12.9%, 11800;12.3%, 22800;11.0%, 47300;12.3%, 112000;10.7%, 212000; 9.74%) in the same manner as in Example 23 and the gene expression was tested. The expression level was highest when the pullulan molecular weight was 47300 (Figure 7).

### EXAMPLE 26

### Change in transfection with DNA according to different spermine introduction rates

The spermine introduction rate was varied in five ways with respect to three types of pullulan with molecular weights of 22800, 47300, and 112000 to prepare a total of 15 spermine-pullulan preparations (N/P ratio 3). The introduction rate of spermine were as follows.

**Table 2**

| Mole ratio | 0.5 | 1.0 | 1.5 | 3.0 | 5.0 |
|---|---|---|---|---|---|
| 22800 | 2.69% | 5.60% | 11.0% | 23.0% | 32.5% |
| 47300 | 1.07% | 5.95% | 12.3% | 20.4% | 32.9% |
| 112000 | 2.19% | 7.35% | 10.7% | 26.3% | 33.1% |

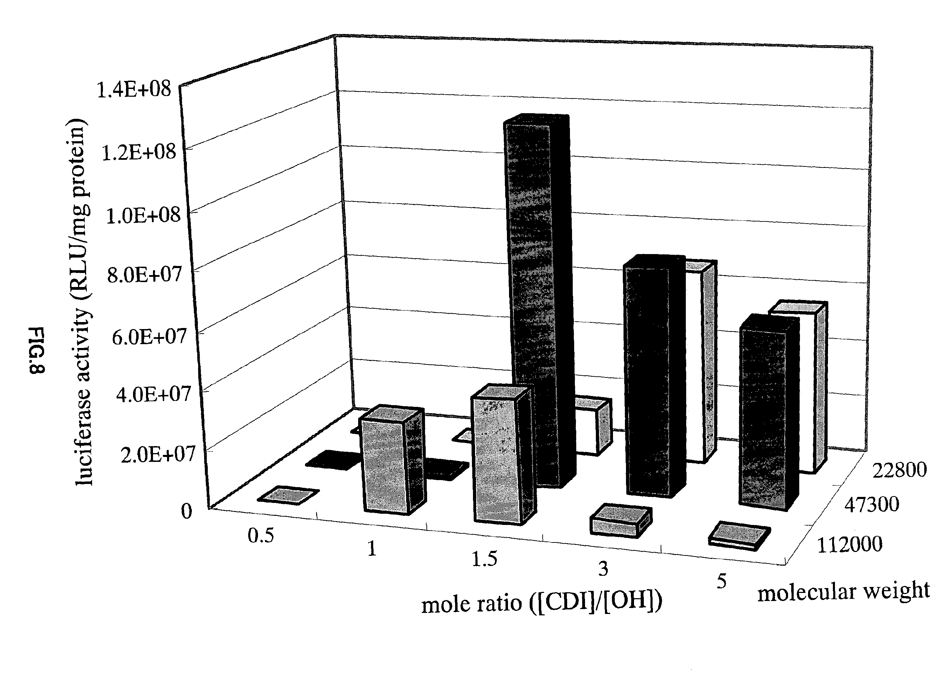

The cells were transfected with pCMV-luc (100 µL) in the same manner as in Example 23, and gene expression was tested. The expression level was highest for the pullulan molecular weight of 22800 with the CDI/OH mole ratio of 3 (spermine introduction rate 23.0%), for the pullulan molecular weight of 47300 with the CDI/OH mole ratio of 1.5 (spermine introduction rate 12.3%), and for the pullulan molecular weight of 112000 with the CDI/OH mole ratio of 1.5 (spermine introduction rate of 10.7%). Among those, the expression level was highest with a pullulan molecular weight of 47300 and a CDI/OH mole ratio of 1.5 (spermine introduction rate 12.3%) (Figure 8).

### EXAMPLE 27

### Transfection of cancer cells with DNA

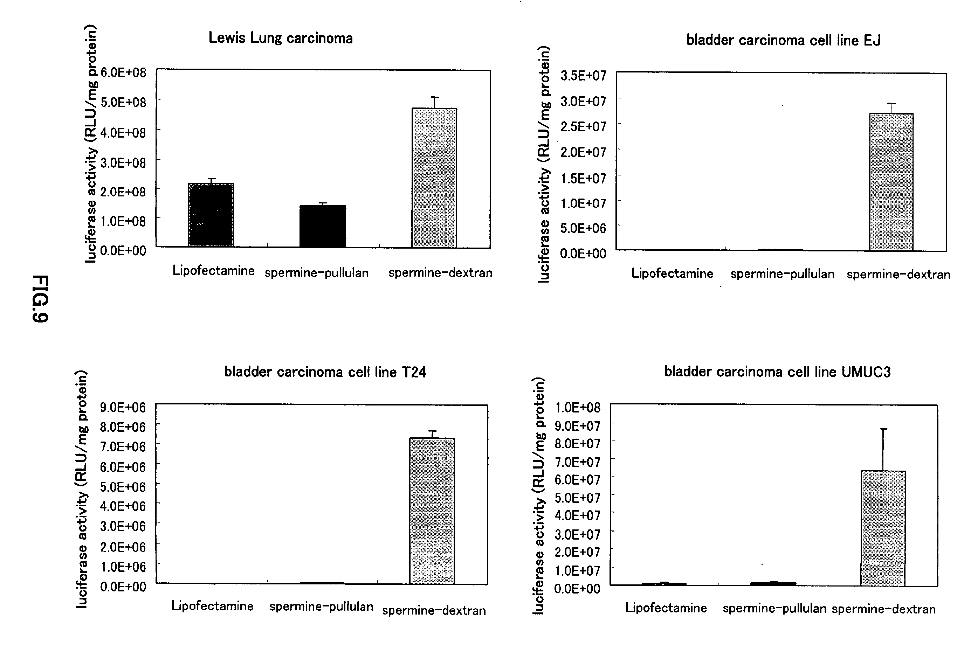

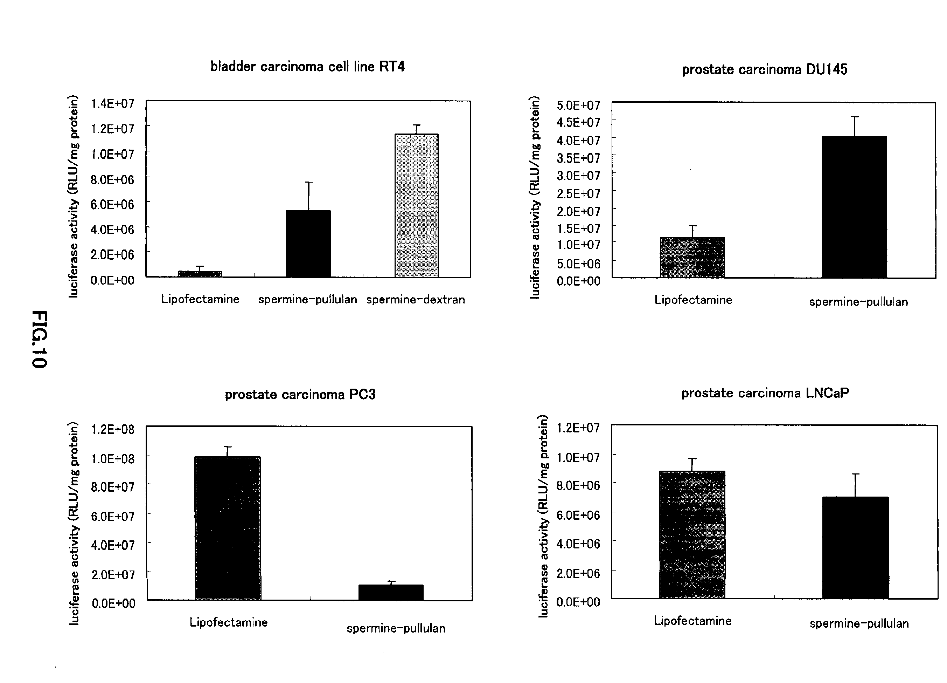

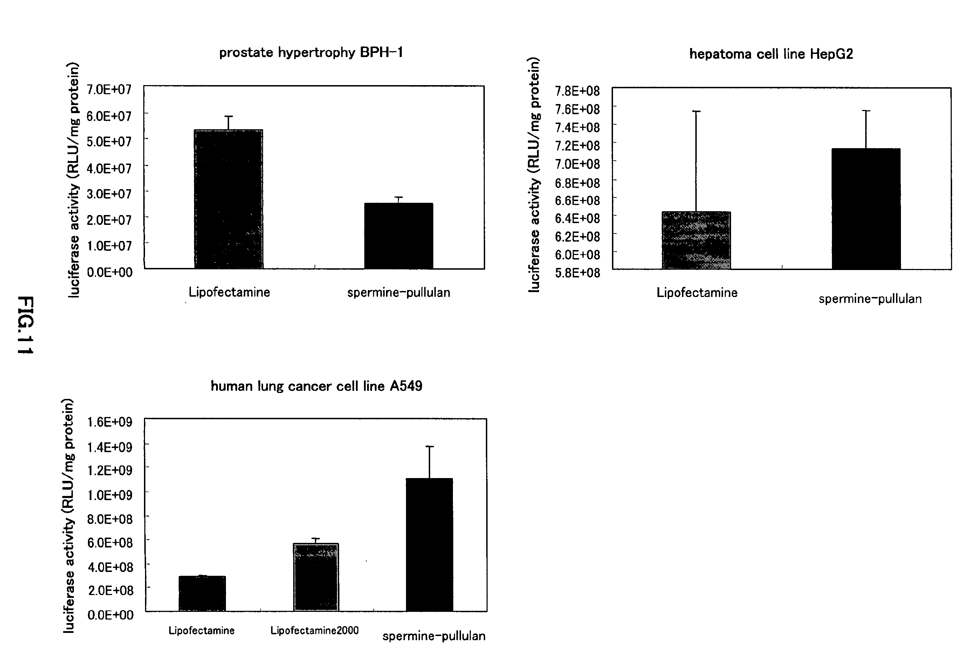

Cancer cells were transfected with an aqueous solution of a polyionic complex consisting of either spermine-pullulan or spermine-dextran and pCMV-luc in the same manner as in Example 23, and the gene expression was tested (Figures 9 to 11). As shown in Figures 9 to 11, when Lewis Lung carcinoma cells were transfected with the nucleic acid, the expression level was significantly higher with spermine-dextran than with LipofectAmine™.

When urinary bladder carcinoma cell lines EJ, T24, UMUC3, and RT4 were transfected with the nucleic acid, almost no transfection was seen with LipofectAmine™, and the gene expression level was significantly higher with the spermine-dextran.

When the prostate carcinoma cell lines DU145, PC3, and LNCaP were transfected with the nucleic acid, the expression level in DU145 cells was significantly higher with the spermine-pullulan than with LipofectAmine™. In PC3 cells the expression level was significantly higher with LipofectAmine™. In LNCaP cells, the expression level was about the same as with LipofectAmine™.

When prostate hypertrophy BPH-1 cells were transfected with the nucleic acid, the expression level with spermine-pullulan was about half the level obtained with LipofectAmine™. When hepatoma cell line HepG2 cells were transfected with the nucleic acid, the expression level with spermine-pullulan was significantly higher than with LipofectAmine™. When human lung cancer cell line A549 cells were transfected with the nucleic acid, the expression level was significantly higher with spermine-pullulan than with LipofectAmine™ and LipofectAmine2000™.

### EXAMPLE 28

### Transfection of stem cells with DNA

Stem cells were transfected with an aqueous solution of a polyionic complex consisting of either spermine-pullulan or spermine-dextran and pCMV-luc in the same manner as in Example 23, and the gene expression was tested.

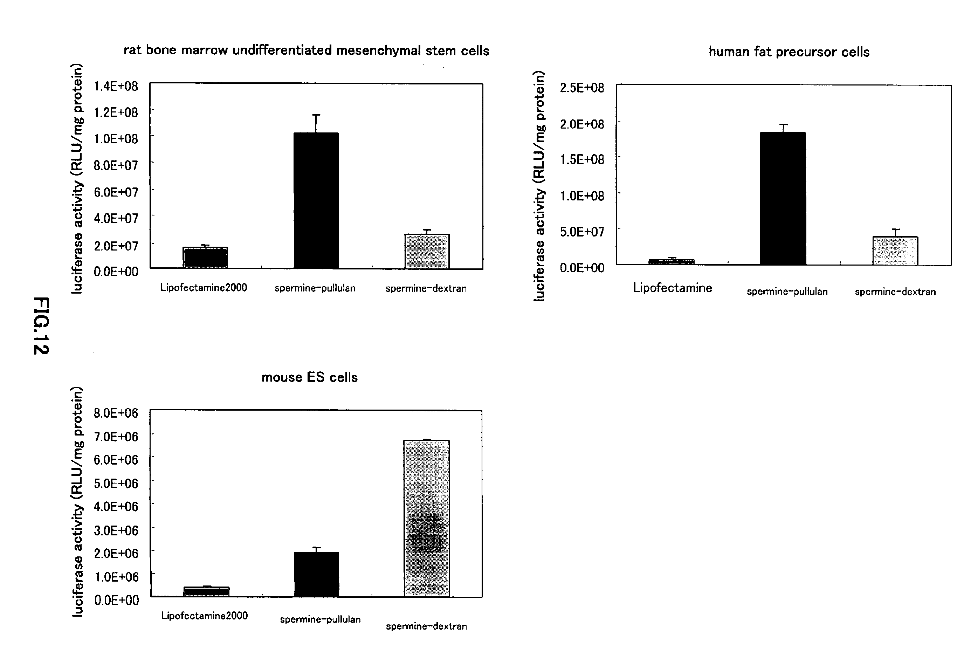

As shown in Figure 12, when rat bone marrow undifferentiated mesenchymal stem cells and human fat precursor cells were transfected with the nucleic acid, the expression level was significantly higher with spermine-pullulan than with LipofectAmine2000™. When mouse ES cells were transfected with the nucleic acid, the expression level with spermine-dextran was significantly higher than with LipofectAmine2000™, and even higher than with spermine-pullulan.

### EXAMPLE 29

### Transfection of primary cultured cells with DNA

Primary cultured cells were transfected with an aqueous solution of a polyionic complex consisting of either spermine-pullulan or spermine-dextran and pCMV-luc in the same manner as in Example 23, and the gene expression was tested.

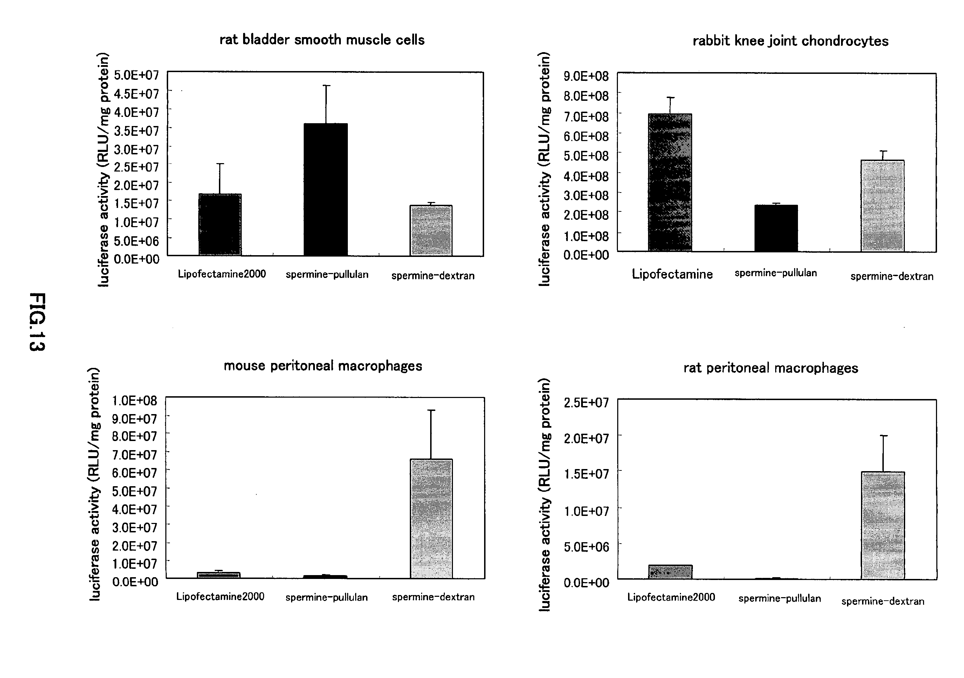

As shown in Figure 13, when rat urinary bladder smooth muscle cells were transfected with the nucleic acid, the expression level was significantly higher with spermine-pullulan than with LipofectAmine2000™. When rabbit knee joint chondrocytes were transfected with the nucleic acid, the expression level with spermine-dextran was about half the level obtained with LipofectAmine2000™. When mouse peritoneal macrophages and rat peritoneal macrophages were transfected with the nucleic acid, the expression level was significantly higher with spermine-dextran than with LipofectAmine2000™.

### EXAMPLE 30

### Transfection of alveolar epithelium type II RLE6TN cells with DNA

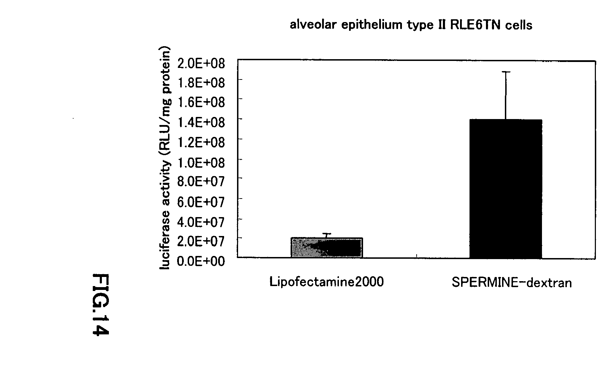

Alveolar epithelium type II RLE6TN cells were transfected with pCMV-luc (100 µL) in the same manner as in Example 23 and the gene expression was tested. As shown in Figure 14, the expression level was significantly higher with spermine-pullulan than with LipofectAmine™.

### EXAMPLE 31

### Transfection of mouse peritoneal macrophages with DNA

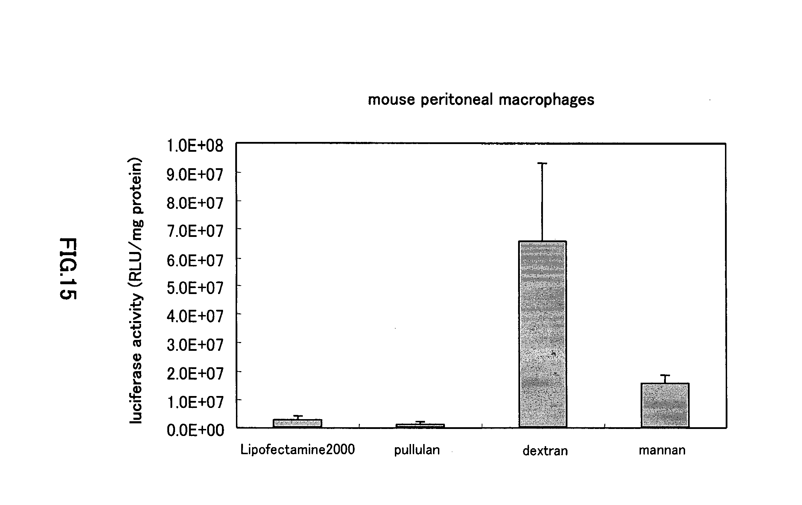

Mouse peritoneal macrophages were transfected with pCMV-luc (100 µL) in the same manner as in Example 23 and the gene expression was tested. As shown in Figure 15, the expression level was significantly higher with spermine-dextran and spermine-mannan than with LipofectAmine2000™.

### EXAMPLE 32

### Transfection of rat peritoneal macrophages with DNA

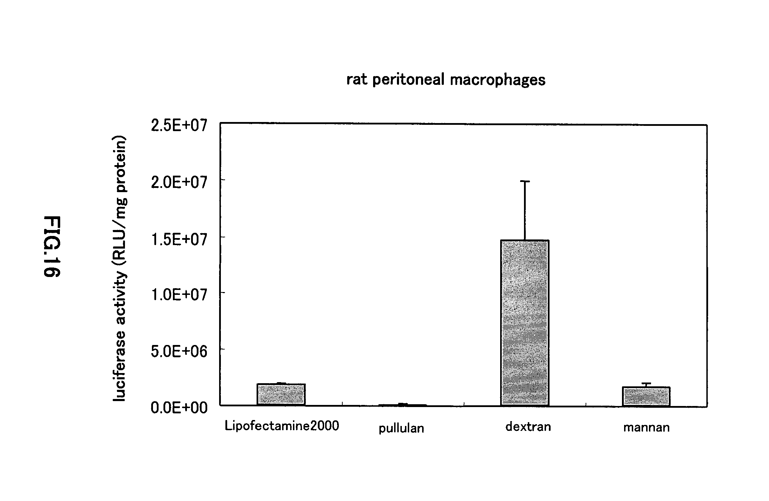

Rat peritoneal macrophages were transfected with pCMV-luc (100 µL) in the same manner as in Example 23 and the gene expression was tested. As shown in Figure 16, the expression level with spermine-mannan was about the same with LipofectAmine2000™, and the expression level with spermine-dextran was significantly higher than with LipofectAmine2000™.

### EXAMPLE 33

### Transfection of mouse ES cells with DNA

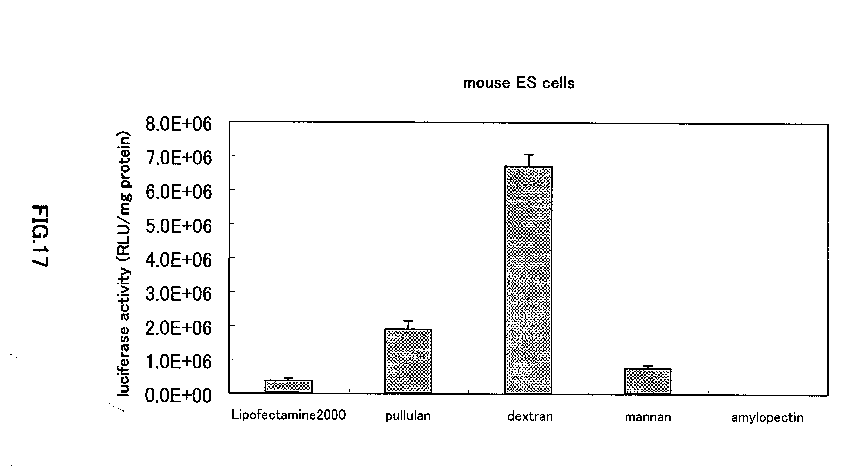

Mouse ES cells were transfected with pCMV-luc (100 µL) in the same manner as in Example 23 and the gene expression was tested. As shown in Figure 17, the expression levels with spermine-mannan and spermine-dextran were significantly higher than with LipofectAmine2000™.

### INDUSTRIAL APPLICABILITY

The composition and method of the present invention for transfecting a cell with a nucleic acid will be useful in gene therapy, cell transplantation therapy, regenerative medicine, and basic biomedical research.

## Claims

1. A composition comprising a polysaccharide for enhancing transfection of a cell with a nucleic acid.

2. The composition according to claim 1, wherein the cell is selected from bone marrow mesenchymal stem cells, nervous system cell lines, adipose tissue stem cells, immunocytes, neurons, chondrocytes, epithelial cells, primary cultured cells, and cancer cells.

3. The composition according to claim 1 or 2, wherein the polysaccharide is a cationized pullulan derivative, a cationized dextran derivative, or cationized mannan derivative.

4. A method for transfecting a cell with a nucleic acid comprising the steps of:
forming a polyionic complex of the nucleic acid and a polysaccharide; and
bringing about uptake of the polyionic complex by the cell.

5. The method according to claim 4, wherein the cell is selected from bone marrow mesenchymal stem cells, nervous system cell lines, adipose tissue stem cells, immunocytes, neurons, chondrocytes, epithelial cells, primary cultured cells, and cancer cells.

6. The method according to claim 4 or 5, wherein the polysaccharide is a cationized pullulan derivative, a cationized dextran derivative, or cationized mannan derivative.
